(19) Europäisches Patentamt
European Patent Office
Office européen des brevets

(11) EP 1 077 058 A2

(12) # EUROPÄISCHE PATENTANMELDUNG

(43) Veröffentlichungstag:
**21.02.2001 Patentblatt 2001/08**

(51) Int. Cl.[7]: **A61K 7/00**, A61K 7/48

(21) Anmeldenummer: **00116201.5**

(22) Anmeldetag: **03.08.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **18.08.1999 DE 19939139**

(71) Anmelder:
**Beiersdorf Aktiengesellschaft**
**20245 Hamburg (DE)**

(72) Erfinder:
- **Gers-Barlag, Heinrich, Dr.**
  **25495 Kummerfeld (DE)**
- **Müller, Anja**
  **23843 Rümpel (DE)**
- **Grotelüschen, Birgit**
  **22459 Hamburg (DE)**

(54) **Emulgatorfreie feindisperse Systeme vom Typ Wasser-in-Öl**

(57) Kosmetische oder dermatologische Zubereitungen, die feindisperse Systeme vom Typ Wasser-in-Öl darstellen, enthaltend

1. eine Ölphase,
2. eine Wasserphase,
3. eine Kombination von

   a) mindestens einem modifizierten Schichtsilikat
   b) und Bornitrid, wobei
   sowohl das oder die modifizierten Schichtsilikate als auch das Bornitrid sowohl hydrophile als auch lipophile Eigenschaften zeigen, also amphiphilen Charakter besitzen und sich an der Grenzfläche Wasser/Öl anordnen und

4. höchstens 0,5 Gew.-% eines oder mehrerer Emulgatoren

sowie
gegebenenfalls weitere kosmetische oder pharmazeutische Hilfs-, Zusatz- und/oder Wirkstoffe enthaltend.

EP 1 077 058 A2

## Beschreibung

Die vorliegende Erfindung betrifft emulgatorfreie feindisperse Systeme vom Typ Wasser-in-Öl, bevorzugt als kosmetische oder dermatologische Zubereitungen.

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester etc.) und mindestens einer Wasserphase (Wasser, Glycerin, Glykole usw.) bestehen, die mit Hilfe von Emulgatoren fein ineinander verteilt werden. Die lipophile Phase einer Körperpflegeemulsion enthält üblicherweise eine Mischung aus Ölen, Fetten und Wachsen, deren Zusammensetzung die produktbestimmenden Eigenschaften wie Hautpflege und Hautgefühl wesentlich beeinflussen soll.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. Sie setzen die Grenzflächenspannung zwischen den Phasen herab, indem sie an der Grenzfläche zwischen beiden Flüssigkeiten angeordnet sind (sie bilden Grenzflächenfilme aus), wodurch dem irreversiblen Zusammenfließen der Tröpfchen entgegengewirkt wird. Zur Stabilisierung von Emulsionen werden häufig Emulgatorgemische verwendet.

Die herkömmlichen Emulgatoren können entsprechend ihrem hydrophilen Molekülteil in ionische (anionische, kationische und amphotere) und nichtionische untergliedert werden:

- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.

Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Kosmetische Zubereitungen werden im wesentlichen zur Hautpflege benutzt. Hautpflege im kosmetischen Sinn ist in erster Linie, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. UV-A-Strahlung (320 bis 400 nm) ist im Hinblick auf die Auslösung photodynamischer, speziell phototoxischer Reaktionen und chronischer Veränderungen der Haut noch weitaus gefährlicher als UV-B-Strahlung. So reicht selbst die UV-A-Strahlung unter ganz normalen Alltagsbedingungen aus, um innerhalb kurzer Zeit die Kollagen- und Elastinfasern zu schädigen. Auch kann der schädigende Einfluß der UV-B-Strahlung durch UV-A-Strahlung noch verstärkt werden.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können kosmetischen bzw. dermatologischen Formulierungen neben UV-Filtersubstanzen zusätzlich Antioxidantien und/oder Radikalfänger zugesetzt werden.

Auch im Bereich der Sonnenschutzformulierungen spielen Emulsionen die entscheidende Rolle. Dabei werden fließfähige Emulsionen bevorzugt, da sie sich leichter auftragen lassen als zähflüssige Cremes. Die meisten Sonnenschutzmittel werden in Wassernähe oder bei sportlicher Betätigung (Schwitzen) angewendet, weshalb der Wasserfestigkeit solcher Formulierungen besondere Bedeutung beizumessen ist. Ein wasserfestes Sonnenschutzmittel schützt den Anwender nicht nur nach dem Baden, sondern bewahrt ihn auch während des Badens vor einem Sonnenbrand.

Um hohe Lichtschutzfaktoren bei gleichzeitiger sehr guter Wasserfestigkeit zu erreichen, sind W/O-Formulierungen von Vorteil. Allerdings weisen W/O-Emulsionen des Standes der Technik häufig unbefriedigende kosmetische Eigenschaften auf. Bei der Anwendung können entsprechende Zubereitungen auf der Haut einen fettigen, glänzenden und zum Teil klebrigen Eindruck hinterlassen und sich - insbesondere auf behaarter Haut - schwierig verteilen lassen. Im Einzelfall können daher derartige Zubereitungen sogar nicht vermarktungsfähig sein, da sie vom Verbraucher nicht akzeptiert bzw. negativ beurteilt werden.

Eine Aufgabe der vorliegenden Erfindung war daher, den Nachteilen des Standes der Technik Abhilfe zu schaffen und Zubereitungen vom Typ Wasser-in-Öl zu finden, welche beispielsweise die Formulierung hoher Lichtschutzfaktoren bei gleichzeitiger sehr guter Wasserfestigkeit erlauben, welche aber auf der Haut keinen schmierigen oder klebrigen Eindruck hinterlassen.

Kosmetische Zubereitungen werden ferner auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, daß bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, daß feinstverteilte Feststoffteilchen eine zusätzliche stabilisierende Wirkung auf eine Emulsion haben. Es kommt zu einer Anreicherung des festen Stoffes an der Phasengrenze Öl/Wasser in Form einer Schicht, durch die ein Zusammenfließen der dispersen Phasen verhindert wird. Von wesentlicher Bedeutung sind dabei nicht die chemischen, sondern die Oberflächeneigenschaften der Feststoffpartikel.

Eine relativ neue technische Entwicklung ist es, kosmetische oder dermatologische Zubereitungen nur durch feinstverteilte Feststoffteilchen zu stabilisieren. Solche „emulgatorfreien" Emulsionen werden nach ihrem Erfinder auch als Pickering-Emulsionen bezeichnet.

Grundlegende Untersuchungen haben gezeigt, daß ein Charakteristikum für eine Pickering-Emulsion ist, daß die Feststoffpartikel an der Grenzfläche zwischen den beiden flüssigen Phasen angeordnet sind und dort sozusagen eine mechanische Barriere gegen die Vereinigung der Flüssigkeitströpfen bilden.

Eine Möglichkeit, eine Feststoffstabilisierung in einer kosmetischen oder dermatologischen Zubereitung vorzunehmen, ist nach May-Alert (*Pharmazie in unserer Zeit*, *15*. *Jahrg*. *1986*, *Nr*. *1*, *1-7*) beispielsweise, Emulgatorgemische zu verwenden, die sowohl anionische als auch kationische Tenside enthalten. Da beim Zusammengeben von Anion- und Kationtensiden immer unlösliche, elektroneutrale Verbindungen ausfallen, läßt sich durch gezieltes Ausfällen dieser neutralen Tenside in der Grenzfläche Öl/Wasser eine zusätzliche Feststoffstabilisierung im Sinne einer Pikkering-Emulsion erreichen.

Ferner beschreibt die WO-Schrift WO-98/42301 emulgatorfreie feindisperse Systeme vom Typ Wasser-in-Öl, welche durch den Zusatz mikronisierter, anorganischer Pigmente aus der Gruppe der Metalloxide stabilisiert werden.

Allerdings konnte der Stand der Technik nicht den Weg zur vorliegenden Erfindung weisen.

Eine weitere Aufgabe der vorliegenden Erfindung war daher, kosmetische und dermatologische Grundlagen für kosmetische und dermatologische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen oder Deodorantien und insbesondere Lichtschutzmittel geschaffen werden sowie zum Beispiel auch Zubereitungen gegen Akne und andere Hauterscheinungen.

Eine weitere Aufgabe der vorliegenden Erfindung war, den Stand der Technik um kosmetische oder dermatologische Zubereitungen zu bereichern, in welchen auf jeglichen Einsatz von Emulgatoren herkömmlicher Art verzichtet werden kann.

Erstaunlicherweise werden alle diese Aufgabe gelöst durch
kosmetische oder dermatologische Zubereitungen, die feindisperse Systeme vom Typ Wasser-in-Öl darstellen, enthaltend

1. eine Ölphase,
2. eine Wasserphase,
3. eine Kombination von

    a) mindestens einem modifizierten Schichtsilikat
    b) und Bornitrid, wobei
    sowohl das oder die modifizierten Schichtsilikate als auch das Bornitrid sowohl hydrophile als auch lipophile Eigenschaften zeigen, also amphiphilen Charakter besitzen und sich an der Grenzfläche Wasser/Öl anordnen und

4. höchstens 0,5 Gew.-% eines oder mehrerer Emulgatoren

sowie
gegebenenfalls weitere kosmetische oder pharmazeutische Hilfs-, Zusatz- und/oder Wirkstoffe enthaltend.

Es ist erfindungsgemäß besonders vorteilhaft, wenn die Zubereitungen deutlich weniger als 0,5 Gew-% eines oder mehrerer Emulgatoren enthalten. Ganz besonders bevorzugt sind erfindungsgemäße Zubereitungen, welche gänzlich frei von Emulgatoren im herkömmlichen Sinn sind.

Die erfindungsgemäßen Zubereitungen stellen Abmischungen aus Ölen bzw. öllöslichen Substanzen und Wasser bzw. wasserlöslichen Komponenten dar, die durch den Zusatz der erfindungsgemäßen Kombination stabilisiert werden und keinen Emulgator im herkömmlichen Sinn enthalten müssen.

Die erfindungsgemäßen Zubereitungen sind in jeglicher Hinsicht überaus befriedigende Präparate, die erstaunlicherweise hervorragende kosmetische Eigenschaften zeigen, auf der Haut keinen schmierigen oder klebrigen Eindruck hinterlassen und sich durch eine ausgezeichnete Hautverträglichkeit auszeichnen.

Silikate sind Salze und Ester (Kieselsäureester) der Orthokieselsäure [$Si(OH)_4$] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus $[SiO_4]^{4-}$-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes Ionenaustausch-Vermögen besitzen und Silizium gegen Aluminium und dieses wiederum gegen Magnesium, $Fe^{2+}$, $Fe^{3+}$, Zn und dergleichen ausgetauscht werden kann. Die daraus möglicherweise resultierende negative Ladung der Schichten wird in der Regel durch Kationen, insbesondere durch $Na^+$ und $Ca^{2+}$ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate, welche in einer erfindungsgemäßen Kombination mit Bornitrid eingesetzt werden können, sind beispielsweise solche, deren größte Ausdehnungsrichtung im unmodifizierten und ungequollenen Zustand im Mittel eine Länge von weniger als 10 µm hat. Beispielsweise können die mittleren Ausdehnungen der verwendeten modifizierten Schichtsilikatpartikel bei 1000 nm x 100 nm x 1 nm und darunter liegen. Die effektive Größe der modifizierten Schichtsilikatpartikel in einer kosmetischen oder dermatologischen Formulierung hängt selbstverständlich von der Menge an eingelagerten Substanzen ab.

Vorteilhafte modifizierte Schichtsilikate im Sinne der vorliegenden Erfindung sind beispielsweise modifizierte Smektite (Smectite). Smektite sind stets sehr feinkörnige (meist < 2 mm), überwiegend als lamellenförmige, moosartige oder kugelförmige Aggregate vorkommende Dreischicht-Tonminerale (2:1 - Schichtsilikate), in denen eine zentrale Schicht aus oktaedrisch koordinierten Kationen sandwichartig von 2 Schichten aus $[(Si,Al)O_4]$-Tetraedern umgeben ist. Smektite werden idealisiert durch die folgende Strukturformel beschrieben, worin weiß ausgefüllte Kreise

Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

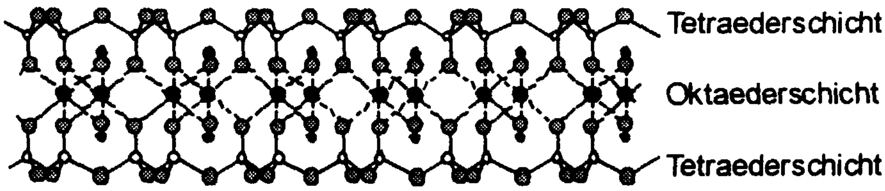


Vorteilhafte modifizierte Smektite sind z. B. modifizierte Montmorillonite. Montmorillonite werden durch die angenäherte chemische Formel $Al_2[(OH)_2/Si_4O_{10}] \cdot n\ H_2O$ bzw. $Al_2O_3 \cdot 4\ SiO_2 \cdot H_2O \cdot n\ H_2O$ beschrieben und stellen zu den dioktaedrischen Smektiten gehörende Tonmineralien dar.

Besonders vorteilhaft im Sinne der vorliegenden Erfindung sind ferner beispielsweise modifizierte Hektorite. Hektorite gehören zu den Smektiten und haben die angenäherte chemische Formel $M^+_{0,3}(Mg_{2,7}Li_{0,3})[Si_4O_{10}(OH)_2]$, worin $M^+$ meist $Na^+$ darstellt.

Vorteilhaft im Sinne der vorliegenden Erfindung sind ferner modifizierte Bentonite. Bentonite sind Tone und Gesteine, die Smektite, vor allem Montmorillonit, als Hauptminerale enthalten. Die „Roh"-Bentonite sind entweder Calcium-Bentonite (in Großbritannien als Fuller-Erden bezeichnet) oder Natrium-Bentonite (auch: Wyoming-Bentonite).

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hectoriten, die durch Ionenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden:

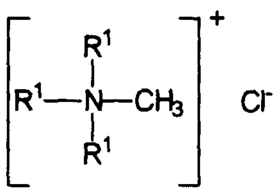

worin

die Reste $R^1$ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Die Gesamtmenge an einem oder mehreren modifizierten Schichtsilikaten in den fertigen kosmetischen oder dermatologischen Zubereitungen wird vorteilhaft aus dem Bereich von 0,05 bis 5,0 Gew.-%, bevorzugt 0,1 bis 1,0 Gew.-% gewählt, bezogen auf das Gesamtgewicht der Zubereitungen.

Vorteilhafte Bornitride, welche in einer erfindungsgemäßen Kombination mit einem oder mehreren modifizierten Schichtsilikaten eingesetzt werden können, sind beispielsweise die im folgenden aufgelisteten Bornitride:

| Handelsname | Erhältlich bei |
|---|---|
| Boron Nitride Powder | Advanced Ceramics |
| Boron Nitride Powder | Sintec Keramik |
| Ceram Blanche | Kawasaki |
| HCST Boron Nitride | Stark |
| Très BN® | Carborundum |
| Wacker-Bornitrid BNP | Wacker-Chemie |

Vorteilhaft ist es, den mittleren Partikeldurchmesser der verwendeten Bornitridpartikel kleiner als 20 µm, besonders vorteilhaft kleiner als 15 µm zu wählen.

Pickering-Emulsionen im Sinne der vorliegenden Erfindung werden ebenfalls vorteilhaft durch Kombinationen von einem oder mehreren modifizierten Schichtsilikaten mit Bornitridpartikeln stabilisiert, die oberflächlich wasserabweisend behandelt („gecoatet") sind, wobei gleichzeitig der amphiphile Charakter gebildet werden bzw. erhalten bleiben soll.

Eine vorteilhafte Beschichtung der Bornitridpartikel besteht aus Dimethylpolysiloxan (auch: Dimethicone), einem Gemisch vollmethylierter, linearer Siloxanpolymere, die endständig mit Trimethylsiloxy-Einheiten blockiert sind. Vorteilhaft sind beispielsweise die bei der Firma Carborundum unter der Handelsbezeichnung Très BN® UHP 1106 erhältlichen, mit Dimethicone behandelten Bornitridpartikel.

Vorteilhaft ist ferner eine Beschichtung der Bornitridpartikel mit Polymethylhydrogensiloxan, einem linearen Polysiloxan, welches auch als Methicone bezeichnet wird. Vorteilhafte, mit Methicone behandelte Bornitridpartikel sind beispielsweise die bei der Firma Carborundum unter der Handelsbezeichnung Très BN® UHP 1107 erhältlichen.

Die Gesamtmenge an Bornitrid (eine oder mehrere Verbindungen) in den fertigen kosmetischen oder dermatologischen Zubereitungen wird vorteilhaft aus dem Bereich von 0,1 bis 5,0 Gew.-%, bevorzugt 0,5 bis 2,0 Gew.-% gewählt, bezogen auf das Gesamtgewicht der Zubereitungen.

Obwohl es besonders bevorzugt ist, die erfindungsgemäßen Zubereitungen nur durch den Zusatz der erfindungsgemäßen Kombination zu stabilisieren, kann es ferner vorteilhaft sein, weitere amphiphile Pigmente einzusetzen, welche gegebenenfalls auch zur Stabilisierung der Pickering-Emulsionen beitragen können.

Solche Pigmente sind beispielsweise mikronisierte, anorganische Pigmente, die gewählt werden aus der Gruppe der amphiphilen Metalloxide, insbesondere aus der Gruppe Titandioxid, Zinkoxid, Siliciumdioxid bzw. Silicate (z. B. Talkum), wobei die Metalloxide sowohl einzeln als auch im Gemisch vorliegen können. Dabei ist es im wesentlichen unerheblich, in welcher der gegebenenfalls natürlich vorkommenden Modifikationen die verwendeten amphiphilen Metalloxide vorliegen.

Vorteilhaft ist es, den mittleren Partikeldurchmesser dieser Pigmente zwischen 1 nm und 200 nm, besonders vorteilhaft zwischen 5 nm und 100 nm zu wählen.

Vorteilhaft im Sinne der vorliegenden Erfindung ist es, neben der erfindungsgemäßen Kombination unbehandelte, nahezu reine Pigmentpartikel einzusetzen, insbesondere solche, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Erfindungsgemäß vorteilhaft ist es ferner, anorganische Pigmente einzusetzen, die oberflächlich wasserabweisend behandelt („gecoatet") sind, wobei gleichzeitig ein amphiphiler Charakter dieser Pigmente gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Ein solches Verfahren, das im folgenden am Beispiel von Titandioxid beschrieben wird, besteht beispielsweise darin, daß die hydrophobe Oberflächenschicht nach einer Reaktion gemäß

$$n\ TiO_2 + m\ (RO)_3\ Si\text{-}R' \rightarrow n\ TiO_2\ (\text{oberfl.})$$

erzeugt wird. n und m sind dabei nach Belieben einzusetzende stöchiometrische Parameter, R und R' die gewünschten organischen Reste. Besonders vorteilhaft sind $TiO_2$-Pigmente, beispielsweise die mit Aluminiumstearat beschichteten, unter der Handelsbezeichnung MT 100 T bei der Firma TAYCA erhältlichen.

Eine weitere vorteilhafte Beschichtung der zusätzlichen Pigmente besteht aus Dimethylpolysiloxan (auch: Dimethicone), einem Gemisch vollmethylierter, linearer Siloxanpolymere, die endständig mit Trimethylsiloxy-Einheiten blockiert sind. Besonders vorteilhafte weitere Pigmente sind im Sinne der vorliegenden Erfindung Zinkoxid-Pigmente, die auf diese Weise beschichtet werden.

Vorteilhaft ist ferner, wenn die neben der erfindungsgemäßen Kombination verwendeten anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel beschichtet sind, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhafte Kombinationspartner sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaft im Sinne der vorliegenden Erfindung ist es ferner, neben der erfindungsgemäßen Kombination eine Mischung verschiedener anorganischer, amphiphiler Pigmenttypen einzusetzen, sowohl Mischungen innerhalb eines Kristalls, beispielsweise als Eisenmischoxid oder Talkum (Magnesiumsilicat), als auch Mischungen mehrerer Metalloxidtypen innerhalb einer Zubereitung. Besonders vorteilhaft sind Magnesiumsilicate, beispielsweise die unter der Handelsbezeichnung Talkum Micron bei der Firma Grolmann erhältlichen.

Die erfindungsgemäße Kombination kann ferner vorteilhaft beispielsweise mit Titandioxidpigmenten kombiniert werden, die mit Octylsilanol beschichtet sind, und/oder mit Siliciumdioxidpartikeln, die oberflächlich wasserabweisend behandelt sind. Geeignete Siliciumdioxidpartikel sind beispielsweise sphärische Polyalkylsilsesquioxan-Partikel wie sie in der Europäischen Offenlegungsschrift 0686 391 erwähnt werden. Solche Polyalkylsilsesquioxan-Partikel sind beispielsweise unter den Handelsbezeichnungen Aerosil R972 und Aerosil 200V bei der Firma Degussa erhältlich.

Weiter vorteilhaft ist es, wenn neben der erfindungsgemäßen Kombination mikrofeine Polymerpartikel eingesetzt werden, welche in der Zubereitung in Form von Feststoffen vorliegen. Günstig im Sinne der vorliegenden Erfindung sind beispielsweise Polycarbonate, Polyether, Polyethylen, Polypropylen, Polyvinylchlorid, Polystyrol, Polyamide, Polyacrylate und dergleichen mehr.

Erfindungsgemäß geeignet sind beispielsweise mikrofeine Polyamid-Partikel, insbesondere die unter der Handelsbezeichnung SP-500 bei der Firma TORAY erhältlichen. Ferner vorteilhaft sind Polyamid 6- (auch: Nylon 6) bzw. Polyamid 12- (auch: Nylon 12) Partikel. Polyamid 6 ist das aus ε-Aminocapronsäure (6-Aminohexansäure) oder ε-Caprolactam aufgebaute Polyamid [Poly(ε-caprolactam)], und Polyamid 12 ist ein Poly(ε-laurinlactam) aus ε-Laurinlactam. Vorteilhaft im Sinne der vorliegenden Erfindung sind beispielsweise Orgasol® 1002 (Polyamid 6) und Orgasol® 2002 (Polyamid 12) von der Firma ELF ATOCHEM.

Weitere mikrofeine Polymerpartikel, welche vorteilhaft neben der erfindungsgemäßen Kombination verwendet werden können, sind mikrofeine Polymethacrylate. Solche Partikel sind beispielsweise unter der Handelsbezeichnung POLYTRAP® bei der Firma DOW CHEMICAL erhältlich.

Es ist insbesondere vorteilhaft, wenngleich nicht zwingend, wenn die weiteren mikrofeinen Polymerpartikel oberflächlich beschichtet sind. Diese Oberflächenbehandlung kann dann bestehen, daß die Polymerpartikel nach an sich bekannten Verfahren mit einer dünnen hydrophilen Schicht versehen werden. Vorteilhafte Beschichtungen bestehen beispielsweise aus Titandioxid ($TiO_2$), Zirkoniumdioxid ($ZrO_2$) oder auch weiteren Polymeren, wie beispielsweise Polymethylmethacrylat. Besonders vorteilhafte mikrofeine Polymerpartikel im Sinne der vorliegenden Erfindung sind beispielsweise die nach dem in der US-Patentschrift 4,898,913 beschriebenen Verfahren zur hydrophilen Beschichtung hydrophober Polymerpartikel erhältlichen.

Der mittlere Partikeldurchmesser der verwendeten mikrofeinen Polymerpartikel wird vorzugsweise kleiner als 100 μm, besonders vorteilhaft kleiner als 50 μm gewählt. Dabei ist es im wesentlichen unerheblich, in welcher Form (Plättchen, Stäbchen, Kügelchen etc.) die verwendeten Polymerpartikel vorliegen.

Ferner vorteilhaft ist es, wenn neben der erfindungsgemäßen Kombination amphiphile modifizierte Polysaccharide eingesetzt werden, welche keine verdickenden Eigenschaften zeigen.

Solche amphiphilen Polysaccharide sind beispielsweise durch Umsetzung von Stärke mit mono-, bi- oder polyfunktionellen Reagenzien bzw. Oxidations-Mitteln in weitgehend polymeranalog verlaufenden Reaktionen erhältlich.

Diese Reaktionen basieren im wesentlichen auf Umwandlungen der Hydroxy-Gruppen der Polyglucane durch Veretherung, Veresterung oder selektive Oxidation. Dabei entstehen z. B. sogenannte Stärkeether und Stärkeester der allgemeinen Strukturformel

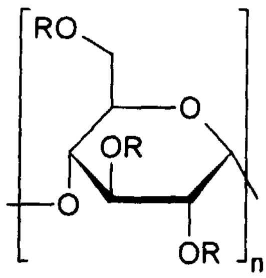

**Strukturformel (I)**

worin R beispielsweise ein Wasserstoff und/oder einen Alkyl- und/oder Aralkylrest (im Fall der Stärkeether) oder ein Wasserstoff und/oder einen organischen und/oder anorganischen Säure-Rest (im Fall der Stärkeester) darstellen kann. Stärkeether und Stärkeester sind vorteilhafte modifizierte Polysaccharide im Sinne der vorliegenden Erfindung.

Besonders vorteilhaft ist es, neben der erfindungsgemäßen Kombination Stärkeether zu verwenden, z. B. solche, die durch Veretherung von Stärke mit Tetramethylolacetylendiharnstoff erhältlich sind und welche als Amylum non mucilaginosum (nicht quellende Stärke) bezeichnet werden.

Insbesondere vorteilhaft sind ferner Stärkeester und/oder deren Salze, beispielsweise Natrium- und/oder Aluminiumsalze niedrigsubstituierter Halbester der Stärke, insbesondere Natrium Stärke n-Octenylsuccinat der Strukturformel (I), worin R sich durch die folgende Struktur auszeichnet

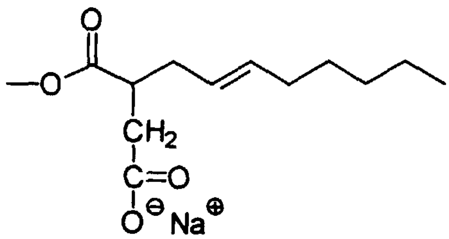

und welches z. B. unter der Handelsbezeichnung Amiogum® 23 bei der Firma CERESTAR erhältlich ist sowie Aluminium Stärke Octenylsuccinat, insbesondere die unter den Handelsbezeichnungen Dry Flo® Elite LL und Dry Flo® PC bei der Firma CERESTAR erhältlichen.

Vorteilhaft ist es, den mittleren Partikeldurchmesser der zusammen mit der erfindungsgemäßen Kombination verwendeten, modifizierten Polysaccharide kleiner als 20 µm, besonders vorteilhaft kleiner als 15 µm zu wählen.

Die Liste der genannten modifizierten Polysaccharide, die vorteilhaft mit der erfindungsgemäßen Kombination kombiniert werden können, soll selbstverständlich nicht limitierend sein. Modifizierte Polysaccharide, die im Sinne der vorliegenden Erfindung vorteilhafte weitere Pigmente darstellen, sind auf zahlreichen, an sich bekannten Wegen, sowohl chemischer als auch physikalischer Natur erhältlich. Zur Herstellung solcher Polysaccharide sind auch neue Wege prinzipiell denkbar. Wesentlich dabei ist, daß die modifizierten Polysaccharide amphiphile Eigenschaften zeigen und daß sie nicht verdickend wirken.

Vorteilhaft in allen vorgenannten Fällen ist es, die Gesamtkonzentration aller weiteren Pigmente größer als 0,05 Gew.-%, besonders vorteilhaft zwischen 0,05 Gew.-% und 30 Gew.-% bezogen auf das Gesamtgewicht der Zubereitungen zu wählen

Die erfindungsgemäßen Pickering-Emulsionen können als Grundlage für kosmetische oder dermatologische Formulierungen dienen. Diese können wie üblich zusammengesetzt sein und beispielsweise zur Behandlung und zur Pflege der Haut, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die

erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Eine erstaunliche Eigenschaft der erfindungsgemäßen Zubereitungen ist, daß diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Stiftzubereitungen mit einem Gehalt an einem oder mehreren Antioxidantien zu versehen, wenn die Zubereitungen einem anderen Zweck dienen sollen, z. B. als Desodorantien oder Sonnenschutzmittel.

Besonders vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine, z. B. Ascorbinsäure und deren Derivate, Vitamine der B- und D-Reihe, sehr günstig das Vitamin $B_1$, das Vitamin $B_{12}$ das Vitamin $D_1$, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaensäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Die Liste der genannten Wirkstoffe bzw. Wirkstoffkombinationen, die in den erfindungsgemäßen Pickering-Emulsionen verwendet werden können, soll selbstverständlich nicht limitierend sein.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen enthalten, insbesondere der Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure:

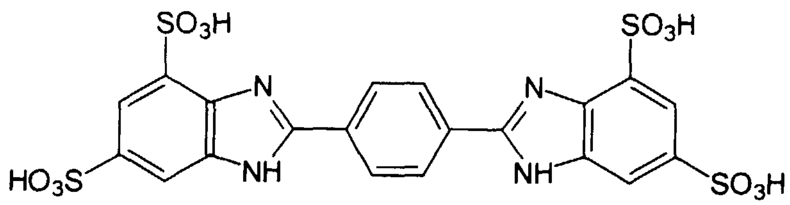

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz:

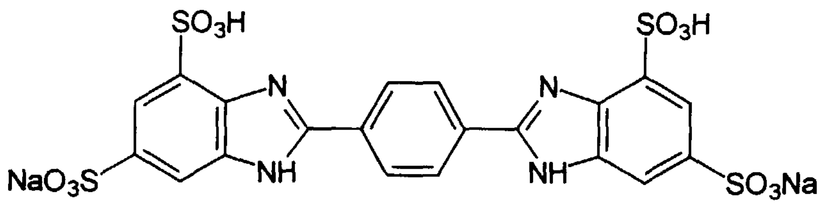

sowie das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

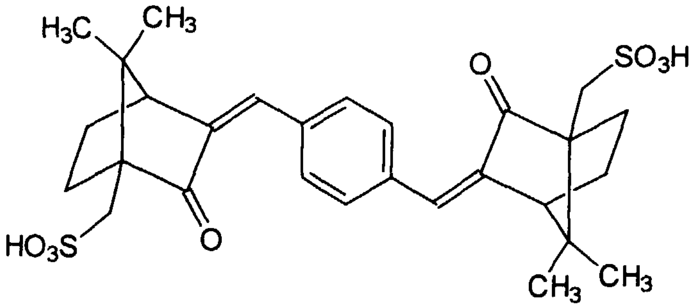

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur:

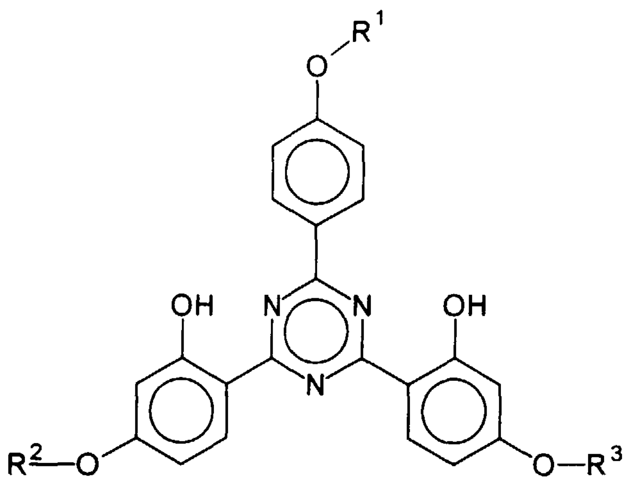

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist, und das 4,4',4"-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch andere UV-Filtersubstanzen beschrieben, welche das Strukturmotiv

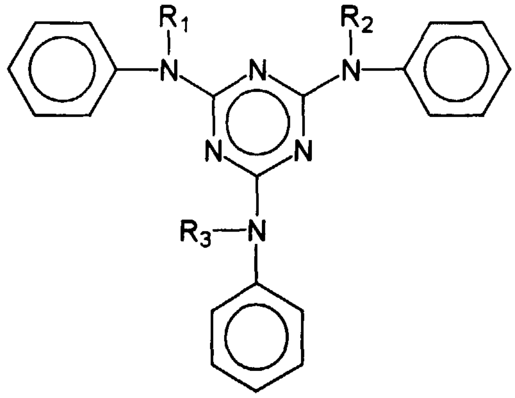

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

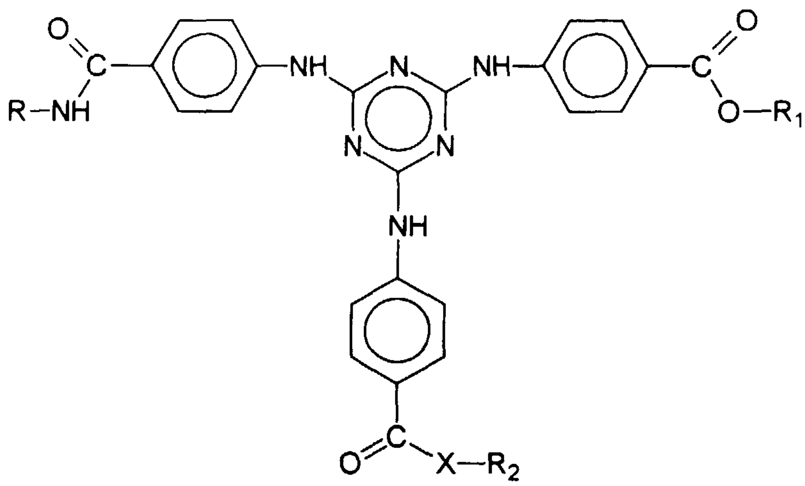

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

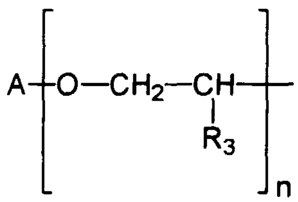

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

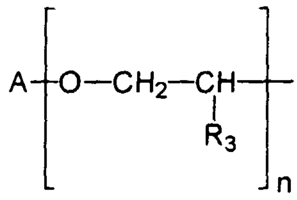

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$- Alkylgruppen,

$R_3$ ein Wasserstoffatom oder eine Methylgruppe darstellt,

n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

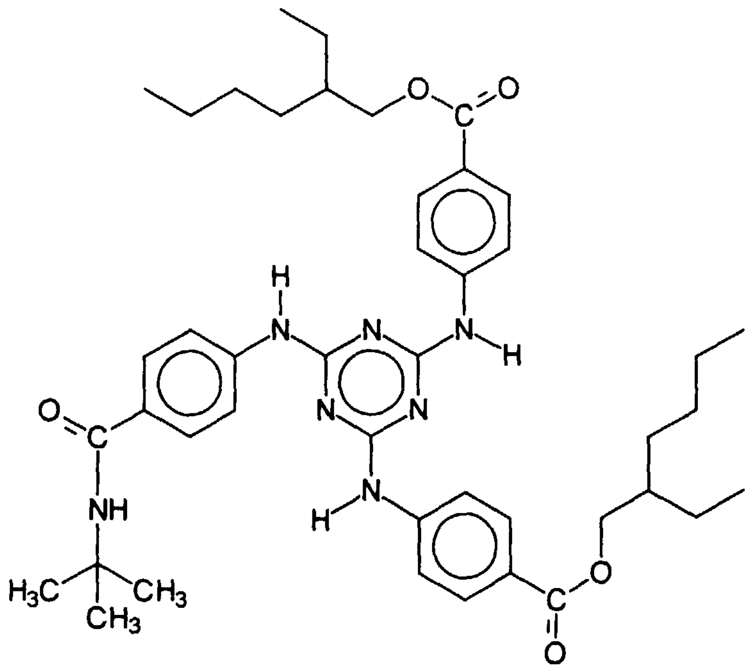

wiedergegeben wird, welche im Folgenden auch als Dioctylbutylamidotriazon (INCI: Dioctylbutamidotriazone) bezeichnet wird und unter der Handelsbezeichnung UVA-SORB HEB bei Sigma 3V erhältlich ist.

Auch in der Europäischen Offenlegungsschrift 775 698 werden bevorzugt einzusetzende Bis-Resorcinyltriazinderivate beschrieben, deren chemische Struktur durch die generische Formel

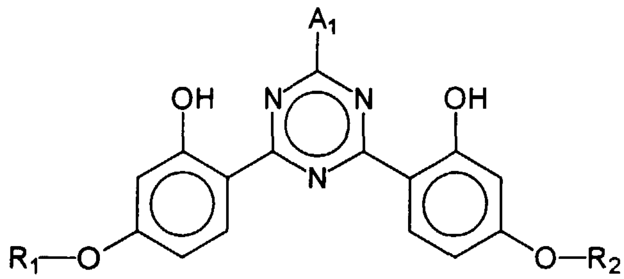

wiedergegeben wird, wobei $R_1$, $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxy-phenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

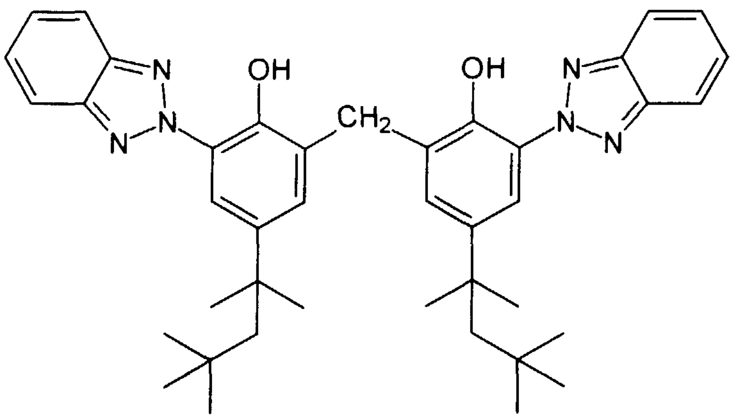

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

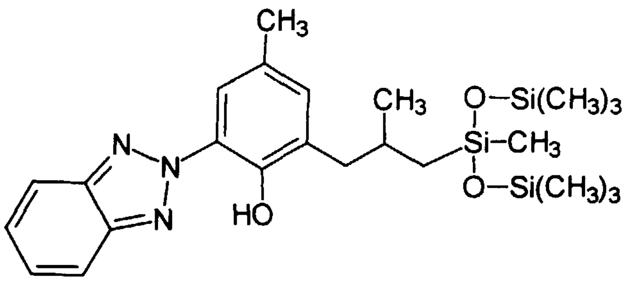

gekennzeichnet ist.

Die UV-B-Filter können öllöslich oder wasserlöslich sein. Vorteilhafte öllösliche UV-B-Filtersubstanzen sind

z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

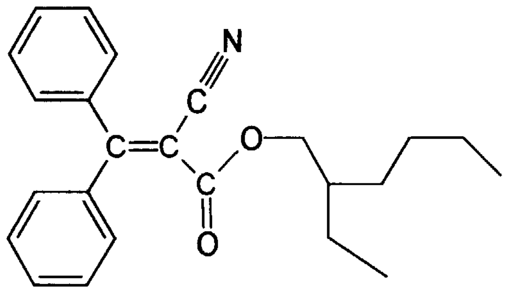

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Die Liste der genannten UV-Filter, die im Sinne der vorliegenden Erfindung eingesetzt werden können, soll selbstverständlich nicht limitierend sein.

Vorteilhaft können erfindungsgemäße Zubereitungen außerdem als Grundlage für kosmetische Desodorantien bzw. Antitranspirantien eingesetzt werden, so daß die vorliegende Erfindung in einer besonderen Ausführungsform Pickering-Emulsionen als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen W/O-Emulsionsstifte eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-196 02 110, DE-196 02 111, DE-196 31 003, DE-196 31 004 und DE-196 34 019 und den Patentschriften DE-42 29 737, DE-42 37 081, DE-43 24 219, DE-44 29 467, DE-44 23 410 und DE-195 16 705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen, die in den erfindungsgemäßen Pickering-Emulsionen verwendet werden können, soll selbstverständlich nicht limitierend sein.

Die erfindungsgemäßen kosmetischen Desodorantien können in Form von aus normalen Behältern auftragbaren wasserhaltigen, kosmetischen Zubereitungen vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Zubereitung.

Die erfindungsgemäßen Stifte sind ferner hervorragende Vehikel für dermatologische Wirkstoffe. Insbesondere eignen sie sich als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Daher ist es vorteilhaft, den erfindungsgemäßen Zubereitungen gegen Akne wirksame Substanzen zuzugeben, die beispielsweise gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden), aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und Calcium-Salze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen (6-[3-(1-Adamantyl)-4-methoxyphenyl]-2-naphthoesäure), Azelainsäure (Nonandisäure), Mesulfen (2,7--Dimethylthianthren, $C_{14}H_{12}S_2$) sowie Aluminiumoxid, Zinkoxid und/oder feinverteilter Schwefel.

Die Menge der Antiaknemittel (eine oder mehrere Verbindungen) in den Zubereitungen beträgt vorzugsweise 0,01 bis 30 Gew.-%, besonders bevorzugt 0,1 bis 20 Gew.-%, insbesondere 1 bis 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

| **Beispiele**: | | | | | | | |
|---|---|---|---|---|---|---|---|
| | 1 | 2 | 3 | 4 | 5 | 6 | 7 |
| Bentone 38 ® | 0,5 | 1 | 0,2 | | 0,2 | | 0,5 |
| Bentone 27 ® | | | 0,3 | 0,7 | | 0,3 | |
| Bornitrid | 0,5 | 2 | 1 | 3 | 5 | 1 | 1 |

(fortgesetzt)

| **Beispiele**: | | | | | | | |
|---|---|---|---|---|---|---|---|
| | **1** | **2** | **3** | **4** | **5** | **6** | **7** |
| Polyglyceryl-2-Dipolyhydroxystearat | | | | | 0,2 | | |
| Cetyl Dimethicon Copolyol | | | | | | 0,3 | |
| PEG-30 Dipolyhydroxystearat | | | | | | | 0,2 |
| Caprylic/Capric Triglycerid | 10 | 5 | | | | 7 | |
| Octyldodecanol | | | 10 | 10 | | 5 | |
| Dicaprylylether | 5 | 5 | 5 | | | 5 | |
| Cetearylisononanoat | | | 2 | | | | |
| Dimethicon | 2 | 2 | 5 | 2 | 5 | | 5 |
| Mineralöl | 5 | 5 | | 10 | 5 | 5 | 2 |
| Isohexadekan | | 2 | 3 | | 5 | | |
| Hydriertes Polyisobuten | 2 | | 3 | 5 | | 5 | |
| Butylen Glycol Dicaprylat/Caprat | | 2 | 5 | 5 | 5 | | 8 |
| $C_{12-15}$ Alkylbenzoat | 7 | 3 | | 3 | 5 | | 10 |
| Vitamen E-Acetat | 0,5 | 0,5 | 0,5 | 0,5 | | 0,5 | |
| Dioctyl Butamido Triazon | | | | | 3 | 1 | |
| Octocrylen | | 5 | | | | 6 | 5 |
| Octyltriazon | 1 | | | | | | |
| Methylbenzyliden Campher | 2 | 4 | | | 2 | | 4 |
| Butyl Methoxydibenzoyl Methan | 2 | | | | 2 | | 2 |
| Titandioxid | 1 | | | 5 | 4 | | 2 |
| Zinkoxid | | | | 5 | | | |
| Aerosil R972 ® | | 0,5 | | | | 0,5 | |
| Konservierung | 0,5 | 0,5 | 0,5 | 0,5 | 0,5 | 0,5 | 0,5 |
| | | | | | | | |
| Glycerin | 3 | 5 | 10 | 10 | 5 | 10 | 5 |
| $MgSO_4$ | | | | | 1 | 1 | |
| NaCl | 0,5 | 1 | 0,2 | | | | 1 |
| Phenylbenzimidazol Sulfonsäure | | | | 2 | | | 4 |
| Natronlauge 45% | | | | 0,8 | | | 1,3 |
| Wasser | ad 100 | ad 100 | ad 100 | ad 100 | ad 100 | ad 100 | ad 100 |

## Patentansprüche

1. Kosmetische oder dermatologische Zubereitungen, die feindisperse Systeme vom Typ Wasser-in-Öl darstellen, enthaltend

1. eine Ölphase,
2. eine Wasserphase,
3. eine Kombination von

    a) mindestens einem modifizierten Schichtsilikat
    b) und Bornitrid, wobei
    sowohl das oder die modifizierten Schichtsilikate als auch das Bornitrid sowohl hydrophile als auch lipophile Eigenschaften zeigen, also amphiphilen Charakter besitzen und sich an der Grenzfläche Wasser/Öl anordnen und

4. höchstens 0,5 Gew.-% eines oder mehrerer Emulgatoren

**2.** Zubereitungen nach Anspruch 1, dadurch gekennzeichnet, daß sie emulgatorfrei sind.

**3.** Zubereitungen nach einem der vorhergehenden Ansprüche, dadurch gekennzeichnet, daß weitere kosmetische oder pharmazeutische Hilfs-, Zusatz- und/oder Wirkstoffe enthalten sind.

**4.** Zubereitungen nach einem der vorhergehenden Ansprüche, dadurch gekennzeichnet, daß der Gehalt an einem oder mehreren modifizierten Schichtsilikaten zwischen 0,05 bis 5,0 Gew.-%, bevorzugt 0,1 bis 1,0 Gew.-% beträgt, bezogen auf das Gesamtgewicht der Zubereitungen.

**5.** Zubereitungen nach einem der vorhergehenden Ansprüche, dadurch gekennzeichnet, daß der Gehalt an Bornitrid (eine oder mehrere Verbindungen) zwischen 0,1 bis 5,0 Gew.-%, bevorzugt 0,5 bis 2,0 Gew.-%, beträgt, bezogen auf das Gesamtgewicht der Zubereitungen.

**6.** Zubereitungen nach einem der vorhergehenden Ansprüche, dadurch gekennzeichnet, daß neben der Kombination aus einem oder mehreren modifizierten Schichtsilikaten und Bornitrid weitere Pigmente enthalten sind, insbesondere modifizierte Polysaccharide und/oder mikrofeine Polymerpartikel und/oder mikronisierte, anorganische Pigmente, die gewählt werden aus der Gruppe der amphiphilen Metalloxide, insbesondere aus der Gruppe Titandioxid, Zinkoxid, Eisenoxide bzw. Eisenmischoxide, Siliciumdioxid bzw. Silicate, wobei die Pigmente sowohl einzeln als auch im Gemisch vorliegen können.

**7.** Zubereitung nach einem der vorhergehenden Ansprüche, dadurch gekennzeichnet, daß sie als UV-Filtersubstanzen unsymmetrisch substituierte s-Triazinderivate, insbesondere 2,4-Bis-{[4-(2-Ethyl-hexyloxy)-2-hydroxy]-phenyl}-6-(4-methoxyphenyl)-1,3,5-triazin und/oder Dioctylbutylamidotriazon, enthält.

**8.** Zubereitung nach einem der vorhergehenden Ansprüche, dadurch gekennzeichnet, daß sie sulfonierte UV-Filter enthält, welche gewählt werden aus der Gruppe Phenylen-1,4-bis-(2-benzimidazyl)-3,3'-5,5'-tetrasulfonsäure und seine Salze, Ben-zol-1,4-di(2-oxo-3-bornylidenmethyl-10-sulfonsäure) und seine Salze, 2-Phenylbenzimidazol-5-sulfonsäure und seine Salze sowie Sulfonsäure-Derivate des 3-Benzylidencamphers und deren Salze.

**9.** Zubereitungen nach einem der vorhergehenden Ansprüche, enthaltend einen oder mehrere Zusatz- oder Wirkstoffe, gewählt aus der Gruppe der Antioxidantien und/oder der Adstringentien und/oder der antimikrobiell wirksamen Stoffe und/oder der gegen Akne wirksamen Stoffe.